# EUROPÄISCHE PATENTSCHRIFT

(11) **EP 2 980 287 B1**
(45) Veröffentlichungstag und Bekanntmachung des Hinweises auf die Patenterteilung: **01.04.2020**
(21) Anmeldenummer: 15175308.4
(22) Anmeldetag: 03.07.2015
(51) Int. Cl.: D01H 13/32, B65H 63/06, G01N 33/36

(54) **MESSWALZE UND VORRICHTUNG ZUM MESSEN EINER DICKE EINES FASERVERBANDES**
MEASURING ROLLER AND DEVICE FOR MEASURING A THICKNESS OF A FIBER SLIVER
ROULEAU MESUREUR ET DISPOSITIF DE MESURE D'UNE ÉPAISSEUR D'UN RUBAN FIBRES

(30) Priorität: 29.07.2014 DE 102014110665
(43) Veröffentlichungstag der Anmeldung: 03.02.2016
(73) Patentinhaber: Rieter Ingolstadt GmbH, 85055 Ingolstadt (DE)
(72) Erfinder: Karalar, Imadettin, 85053 Ingolstadt (DE); Kriegler, Albert, 85290 Geisenfeld (DE); Schmolke, Werner, 85053 Ingolstadt (DE)
(74) Vertreter: Bergmeier, Werner

(56) Entgegenhaltungen:
- EP-A1- 0 478 734
- CN-A- 103 900 867
- CN-Y- 2 229 439
- DE-A1- 3 219 332
- DE-A1-102011 051 552
- DE-C- 491 941
- JP-A- 2001 295 144

## Beschreibung

Die vorliegende Erfindung betrifft eine Vorrichtung zum Messen einer Dicke eines Faserverbandes mit zwei drehbar gelagerten Messwalzen mit Mantelflächen, wobei die Drehachsen der Messwalzen parallel zueinander ausgerichtet sind, und dass der Faserverband zwischen den Mantelflächen der Messwalzen hindurchführbar ist und mit einer Anpressvorrichtung, mittels der der Abstand der Messwalzen zueinander veränderbar ist, und dass damit ein Druck auf den durch die Messwalzen eingeschlossenen Faserverband aufbringbar ist. Des Weiteren betrifft die Erfindung eine Messwalze zur Verwendung in einer Vorrichtung zum Messen einer Dicke eines Faserverbandes, insbesondere an einer Strecke, Karde oder Kämmmaschine, mit einer Oberflächenbeschichtung, und wobei die Messwalze eine Mantelfläche sowie eine Drehachse aufweist.

Bisher bekannt ist ein Messsystem mit glatten Abzugsscheiben. Die Scheiben haben einerseits die Aufgabe, das Faserband zu fördern und andererseits die Dicke des Bandes zu messen. Eine der Scheiben ist beweglich gelagert. Die bewegliche Abzugsscheibe wird mit einem definiert hohen Druck an die starr gelagerte Scheibe gepresst. Die Auslenkung der beweglich gelagerten Scheibe wird als Messsignal ausgewertet. Voraussetzung für ein verwertbares Messsignal ist die definiert hohe Kraft, die auf das Faserband wirken muss und eine sehr hohe Rundlaufgüte der Messwalzen. Nach den Messwalzen wird das Band in ein Rohr gefördert. Dazu muss das Band mittig in das Rohr einlaufen, da sonst kein Abzug möglich ist.

Aus der DE 10 2011 051 552 A1 ist eine Walzenstreckvorrichtung für eine Spinnereimaschine bekannt. Darin wird eine Walzenstreckvorrichtung zum Verziehen wenigstens eines laufenden strangförmigen Stapelfaserverbandes an wenigstens einer Arbeitsstelle einer Spinnereimaschine, insbesondere an einer Spinnmaschine oder an einem Flyer, vorgeschlagen. Dabei ist je Arbeitsstelle ein Verzugsfeld vorgesehen, welches zwischen einem einlaufseitigen Walzenpaar und einem auslaufseitigen Walzenpaar gebildet ist, wobei dem Verzugsfeld eine Faserführungseinrichtung zum Führen des Stapelfaserverbandes zugeordnet ist. Dabei weist die Faserführungseinrichtung eine Faserführungsfläche auf, die wiederum eine dreidimensionale Struktur, insbesondere Orangenhautstruktur oder eine Wabenstruktur, aufweist. Eine derartige dreidimensionale Struktur verhindert ein Anhaften des Stapelfaserverbandes an der Faserführungsfläche durch Adhäsionserscheinungen. Nachteilig hierbei ist jedoch, dass die Orangenhautstruktur oder die Wabenstruktur schwer herzustellen ist.

Aus der EP 0 478 734 B1 ist eine Vorrichtung zur Messung der Dicke eines textilen Faserverbandes bei einem Streckwerk bekannt. Die Vorrichtung weist ein Tastwalzenpaar auf, deren eine Tastwalze im Achsabstand zur anderen Tastwalze entsprechend der Dicke des Faserverbandes veränderbar ist. Dabei ist der gesamte Faserverband zwischen dem Tastwalzenpaar führbar und die im Achsabstand veränderbare Tastwalze ist gegen die andere Tastwalze anpressbar. Nachteilig hierbei ist, dass es bei derartigen Tastwalzen zu Adhäsionserscheinungen zwischen dem Faserverband und den Tastwalzen führen kann, welche ein gerades Austreten des Faserverbandes aus den Tastwalzen verhindert. Dadurch wird ein danach liegendes Ablegen des Faserverbandes in einen dafür vorgesehenen Behälter schwer kontrollierbar. Um die Adhäsionserscheinungen zu verringern, kann beispielsweise der Druck, mit dem eine Tastwalze auf die andere Tastwalze angepresst ist, verringert werden. Dadurch wird jedoch die Messung der Dicke des Faserverbandes verfälscht. Eine Verringerung des Anpressdrucks einer Tastwalze auf die andere Tastwalze entfällt somit.

Insbesondere bei Verarbeitung feiner Bänder kommt es zu den beschriebenen Adhäsionserscheinungen. Das feine Band wird von der glatten Walze mitgeschleppt und ausgelenkt und kann dadurch nicht mehr in das Rohr gefördert werden. Eine Verarbeitung ist nicht mehr möglich. Diesem Effekt konnte bisher nur entgegengewirkt werden, indem man die Anpresskraft auf die beweglich gelagerte Walze soweit reduzierte, dass ein halbwegs gerader Lauf erreicht wurde. Die Reduzierung der Anpresskraft bewirkt aber auch eine Schwächung oder das gänzliche Ausbleiben des Messsignales. Die Überwachung der Qualitätsparameter incl. Off Limit Abstellfunktion des auslaufenden Bandes ist nicht mehr möglich.

Aufgabe der Erfindung ist es somit, eine Vorrichtung und eine Messwalze für die Vorrichtung zum Messen einer Dicke eines Faserverbandes zu schaffen, die leicht herzustellen ist und die Adhäsionserscheinungen zwischen der Messwalze und dem Faserverband verhindert.

Die Aufgabe wird gelöst durch eine Messwalze für eine Vorrichtung zum Messen einer Dicke eines Faserverbandes und einer Vorrichtung zum Messen einer Dicke eines Faserverbandes nach dem unabhängigen Anspruch.

Vorgeschlagen wird eine Vorrichtung zum Messen einer Dicke eines Faserverbandes, mit zwei drehbar gelagerten Messwalzen mit Mantelflächen, wobei die Drehachsen der Messwalzen parallel zueinander ausgerichtet sind, und dass der Faserverband zwischen den Mantelflächen der Messwalzen hindurchführbar ist.

Des Weiteren umfasst die Vorrichtung zum Messen einer Dicke eines Faserverbandes eine Anpressvorrichtung, mittels der der Abstand der Messwalzen zueinander veränderbar ist, und dass damit ein Druck auf den durch die Messwalzen eingeschlossenen Faserverband aufbringbar ist. Beispielsweise ist eine Messwalze fest in der Anpressvorrichtung angeordnet und die andere Messwalze ist gegenüber der festen Messwalze beweglich angeordnet. Die bewegliche Messwalze wird mittels der Anpressvorrichtung mit einem konstanten Druck gegen die feststehende Messwalze angepresst, wobei der Faserverband zwischen diesen beiden Messwalzen hindurchgeführt wird. Bei einer Änderung der Dicke des Faserverbandes ändert sich ebenfalls der Abstand zwischen den beiden Messwalzen. Mittels geeigneter Mittel, beispielsweise eines Sensors, kann der Abstand der beiden Messwalzen gemessen werden, welcher der Dicke des Faserbandes entspricht. Die Dicke des Faserbandes kann dabei als ein Indikator für eine Qualität des Faserbandes herangezogen werden oder dazu benutzt werden, eine Einstellung der Strecke, der Karde oder der Kämmmaschine zu verändern.

Außerdem können die beiden Messwalzen auch als Abzugswalzen genutzt werden. Mittels der Abzugswalzen kann der Faserverband durch eine Strecke, Karde und/oder Kämmmaschine gefördert werden. Die Abzugswalzen ziehen dabei den Faserverband durch die Strecke, Karde oder Kämmmaschine. Beispielsweise kann des Weiteren nach der Vorrichtung zum Messen des Faserverbandes ein Drehteller und darauf folgend eine Kanne angeordnet sein, wobei der Faserverband durch den Drehteller zu der Kanne geleitet wird. In der Kanne wird der Faserverband abgelegt. Der Drehteller kann dabei ein Rohr aufweisen, in das der Faserverband in den Drehteller eingeführt wird.

Erfindungsgemäß weist die Mantelfläche zumindest einer der Messwalzen eine Luftpolster bildende Struktur aus Rillen, Bohrungen und/oder kugelförmigen Erhebungen auf. Mittels der Luftpolster bildenden Struktur der Messwalzen kann eine Adhäsionserscheinung verhindert werden. Die Adhäsionserscheinung würde beispielsweise dazu führen, dass der Faserverband an den Messwalzen haften bleibt und so zumindest teilweise um die Messwalzen herumgewickelt wird. Insbesondere wird durch das Herumwickeln verhindert, dass der Faserverband geradlinig in das Rohr des Drehtellers einläuft. In einem besonders nachteiligen Szenario können die Adhäsionserscheinungen den Faserverband derart weit um die Messwalzen wickeln und ablenken, dass der Faserverband an dem Rohr vorbei läuft. Ein kontrolliertes Ablegen des Faserverbandes in der Kanne ist somit verhindert.

Durch das Verhindern der Adhäsionserscheinung mittels der Rillen, Bohrungen und/oder kugelförmigen Erhebungen ist ein geradliniges Einlaufen des Faserverbandes in das Rohr des Drehtellers gegeben.

Die Messwalzen sind aus einem Material geringer Wärmedehnung ausgebildet und weisen eine Oberflächenbeschichtung auf, die einen Verschleiß der Messwalze verringert. Eine geeignete Oberflächenbeschichtung kann beispielsweise Chrom aufweisen.

Vorgeschlagen wird ferner eine Messwalze für eine Vorrichtung zum Messen einer Dicke eines Faserverbandes, insbesondere an einer Strecke, Karde oder Kämmmaschine. Die Messwalze ist dabei aus einem Material geringer Wärmeausdehnung hergestellt, um bei einer Erwärmung der Messwalze durch Reibung zwischen dieser und dem Faserverband eine Verfälschung der Messung zu verringern.

Des Weiteren weist die Messwalze eine Mantelfläche zur Klemmung eines Faserverbandes sowie eine Drehachse auf.

Erfindungsgemäß weist die Mantelfläche der Messwalze Rillen, Bohrungen und/oder kugelförmigen Erhebungen auf. Durch die Rillen, Bohrungen und/oder kugelförmigen Erhebungen wird eine Kontaktfläche zwischen der Mantelfläche und dem Faserverband verringert. Diese Kontaktfläche wird auch als Wirkfläche bezeichnet. In den Rillen, Bohrungen und/oder zwischen den kugelförmigen Erhebungen bildet sich ein Luftpolster oder Luftkissen aus und/oder Luft kann seitlich durch die Rillen, Bohrungen und/oder zwischen den kugelförmigen Erhebungen in Richtung des Faserverbandes zuströmen. Dabei liegt das Luftkissen zwischen der Mantelfläche und dem Faserverband und verringert so die Adhäsionserscheinung. Durch die Verringerung der Adhäsionserscheinung wird ein Mitschleppen und Auslenken des Faserbandes oder gar ein Wickeln des Faserverbandes in Umfangsrichtung um die Messwalze verringert oder ganz vermieden. Bei einer Vorrichtung in der die Messwalze angeordnet ist, kann ein Vliestrichter und ein Drehteller nachgeordnet sein, der den Faserverband in eine Kanne ablegt. Der Vliestrichter bzw. der Drehteller weist dabei ein Rohr auf, durch das der Faserverband in den Drehteller eingeführt wird. Durch die Verringerung der Adhäsionserscheinung kann der Faserverband vorteilhafterweise geradlinig und insbesondere mittig in das Rohr eingeführt werden. Somit kann der Faserverband kontrolliert in der Kanne abgelegt werden, in welcher der Faserverband für eine Weiterverarbeitung zur Verfügung steht.

Riffelungen bzw. Kanneluren etc. sind in der textilen Verarbeitung grundsätzlich bei anderen Anwendungen seit langem bekannt. Eine normale, bisher bekannte Form der Riffelung würde das Messsignal so sehr verfälschen, das es unbrauchbar wäre. Durch die Art der Riffel erkennt das Mess-System periodische Fehler. Zudem schädigt die benötigte, hohe Anpresskraft das Fasergut. Feine Bänder mit feinen Fasern, die sehr parallel im Faserverband liegen, wie sie z.B. beim Airjet-Prozess gebräuchlich sind, können sehr leicht geschädigt werden. Das hat eine verminderte Garnqualität zur Folge.

Mit den beschriebenen Geometrien der Riffelung wird folgendes erreicht:
- 1.: Gerader Lauf des auslaufenden Faserbandes ohne Reduzierung der Anpresskraft
- 2.: Transport des Bandes ohne Schädigung des Fasergutes
- 3.: Verwertbares Messsignal für die Messung der Band-Qualitätsparameter.

Ohne die beschriebenen Maßnahmen ist insbesondere eine Verarbeitung feiner Bänder nur mit Qualitätsverlusten.

Erfindungsgemäß ist der Abstand der Rillen in Umfangsrichtung zwischen 0,25 mm und 1,25 mm, insbesondere zwischen 0,56 mm und 0,85 mm. Ebenso ist zusätzlich die Rillentiefe zwischen 0,1 mm und 1,0 mm, insbesondere zwischen 0,2 mm und 0,45 mm. Zusätzlich ist es von Vorteil, wenn die Rillen einen Öffnungswinkel von 40° bis 105°, insbesondere von 60° bis 84°, aufweisen. Zusätzlich ist die Rillenbreite zwischen 0,2 mm und 1,0 mm, insbesondere zwischen 0,41 mm und 0,59 mm. Durch verändern der Parameter kann die Größe der Wirkfläche variiert werden. Beispielsweise wird die Wirkfläche größer, wenn der Öffnungswinkel, bei gleichbleibender Anzahl der Rillen, abnimmt. Dabei besteht eine Abhängigkeit von Öffnungswinkel zur Rillenbreite. Wird der Öffnungswinkel kleiner, verringert sich auch die Rillenbreite. Bei gleichbleibender Anzahl und Tiefe der Rillen wird somit die Fläche zwischen den Rillen, welche die Wirkfläche darstellt, größer. Ebenso wird die Wirkfläche größer, wenn der Abstand in Umfangsrichtung zwischen den Rillen größer wird. Dagegen kann das Luftkissen vergrößert werden, wenn die Rillentiefe erhöht wird. Eine Erhöhung der Rillenbreite führt dabei ebenfalls zu einer Vergrößerung des Luftkissens. Dabei sinkt die Adhäsionserscheinung, wenn die Wirkfläche kleiner und/oder das Luftkissen größer wird.

Außerdem kann mit der Geometrie der Rillen eine Beschädigung des Faserverbandes verhindert werden. Wie im Stand der Technik beschrieben, muss bei der Messung des Faserverbandes die Messwalze mit einem definierten, und insbesondere mit einem ausreichend hohen Druck, gegen eine weitere Messwalze gedrückt werden. Dabei können einzelne Fasern des Faserverbandes an den Kanten abgeknickt werden, so dass diese abbrechen und die Qualität des Faserverbandes abnimmt. Weisen jedoch die Rillen beispielsweise eine geringe Breite, z.B. 0,2 mm, auf, können sich die einzelnen Fasern nur wenig in die Rille einbiegen. Dadurch werden die einzelnen Fasern weniger abgeknickt und die Gefahr, dass diese abbrechen ist verringert.

Wenn des Weiteren der Öffnungswinkel der Rillen größer ist, ist eine Kante zwischen der Rille und der Mantelfläche weniger stark ausgebildet, so dass die Gefahr geringer ist, dass die einzelnen Fasern an den Kanten abbrechen.

Ebenfalls ist es von Vorteil, wenn die Anzahl der Rillen in Umfangsrichtung zwischen 50 und 1000, insbesondere zwischen 200 und 400, ist. Der Umfang beträgt zum Beispiel zwischen 100 und 250 mm, vorzugsweise etwa 170 mm. Hierdurch kann die Größe der Wirkfläche variiert werden. Die Anzahl der Rillen in Umfangsrichtung ist dabei abhängig von der Rillenbreite, dem Öffnungswinkel der Rillen, der Rillentiefe und dem Abstand der Rillen in Umfangsrichtung. Daneben ergibt sich aus der Anzahl der Rillen auch ein Teilungswinkel. Dieser berechnet sich aus 360° eines vollen Kreises geteilt durch die Anzahl der Rillen. Somit beträgt der Teilungswinkel zwischen 0,36° und 7,2°, insbesondere zwischen 0,9° und 1,8°. Ebenfalls ergibt sich aus der Anzahl der Rillen eine Teilung. Diese Teilung folgt aus dem Umfang der Messwalze geteilt durch die Anzahl der Rillen. Die Teilung beträgt zwischen 0,1 mm und 5 mm. Eine höhere Anzahl an Rillen führt dabei zu einer Abnahme der Wirkfläche und wiederum zu einer geringeren Adhäsionserscheinung.

Um einen weiteren Parameter zur Verfügung zu stellen, um die Adhäsionserscheinung zu beeinflussen, ist es vorteilhaft, wenn sich zumindest ein Teil der Rillen in einem Winkel von 0° bis 90°, insbesondere in einem Winkel von 16° bis 75°, schneiden. Dabei kann zumindest ein Teil der Rillen axial angeordnet sein, und der andere Teil der Rillen zu den axialen Rillen den Winkel aufweisen. Alternativ ist es auch möglich, dass beide Teile der Rillen schräg über die Mantelfläche der Messwalze verlaufen, sich dabei jedoch noch in dem Winkel schneiden, so dass schräg gegenläufige Rillen ausgebildet sind. Somit sind die Rillen so ausgebildet, dass sie eine Verbindung untereinander aufweisen. Wenn der Winkel 0° beträgt, liegen alle Rillen parallel zueinander. Dabei können die Rillen als Längs-, Quer- und/oder Schrägrillen ausgebildet sein, d.h. die Rillen sind in Axialrichtung, Umfangsrichtung oder schräg auf der Mantelfläche der Messwalze angeordnet. Wenn sich die Rillen untereinander schneiden führt dies dazu, dass sich das Luftkissen zwischen den jeweiligen Rillen verteilen kann und sich so schneller ausbildet, was die Adhäsionserscheinung verringert.

Beispielsweise wenn die Rillen in Umfangsrichtung angeordnet sind, kann die Gefahr, dass die einzelnen Fasern abbrechen, verhindert werden. Bei dieser Anordnung der Rillen sind die einzelnen Fasern parallel zu den Rillen angeordnet, so dass sich die einzelnen Fasern in den Rillen einlagern können, wobei diese nicht abknicken können.

Der Durchmesser der Bohrungen auf der Mantelfläche ist, erfindungsgemäß, zwischen 0,1 mm und 1,25 mm, insbesondere zwischen 0,25 mm und 0,75 mm. Durch größere Bohrungen wird ebenfalls die Wirkfläche verringert und das Luftkissen zwischen der Mantelfläche der Messwalze und dem Faserverband wird vergrößert, was positiven Einfluss auf die Adhäsionserscheinung hat. Dabei ist es jedoch nicht notwendig, dass alle Bohrungen den gleichen Durchmesser aufweisen. Da bei jeder Ausrichtung von runden Bohrungen ein Zwischenraum entsteht, ist es denkbar, dass in diesem Zwischenraum kleinere Bohrungen angeordnet sind. Auch vorteilhaft ist, wenn die Bohrungen auf der Mantelfläche, insbesondere in den Seitenbereichen, schräg eingebohrt sind. Dies führt zu einer weiteren Verringerung der Wirkfläche und damit zu einer Verringerung der Adhäsionserscheinung. Auch hier können beispielsweise kleinere Bohrungen die Gefahr verringern, dass die einzelnen Fasern im Faserverband abgeknickt werden und so die Qualität des Faserverbandes beeinträchtigen.

Erfindungsgemäß ist die Tiefe der Bohrung zwischen 0,1 mm und 1,0 mm, insbesondere zwischen 0,2 mm und 0,45 mm, ist. Durch eine größere Tiefe der Bohrungen wird so ebenfalls das Luftkissen größer, was wiederum zu einer Verringerung der Adhäsionserscheinung führt. Bei einer geringeren Tiefe der Bohrung können die einzelnen Fasern nicht so weit abgeknickt werden, dass diese abbrechen können. Dagegen sind die einzelnen Fasern bei einer größeren Tiefe der Bohrungen "weicher" in die Bohrungen gebogen, so dass die Gefahr verringert ist, dass die Fasern an der Kante zwischen Bohrung und Mantelfläche abbrechen.

Ebenso ist es vorteilhaft, wenn die Anzahl der Bohrungen auf der Mantelfläche zwischen 1000 und 25000, insbesondere zwischen 5000 und 15000, ist. Durch eine höhere Anzahl an Bohrungen sinkt die Größe der Wirkfläche, was ebenfalls die Adhäsionserscheinung verringert.

Die kugelförmigen Erhebungen weisen, erfindungsgemäß, einen Radius zwischen 0,1 µm und 10 µm, insbesondere zwischen 0,6 µm und 4 µm, auf. Das Luftkissen liegt hier zwischen den Erhebungen. Ähnlich zu den Bohrungen ist es auch bei den kugelförmigen Erhebungen, dass bei jeder Ausrichtung von kugelförmigen Erhebungen Zwischenräume entstehen. In diesen Zwischenräumen können dann natürlich auch kleinere kugelförmige Erhebungen angeordnet werden. Im Allgemeinen ist es daher denkbar, dass die kugelförmigen Erhebungen auf der Mantelfläche unterschiedliche Größen aufweisen und/oder auch zufällig oder beliebig über der Mantelfläche verteilt sind. Dabei vergrößert sich das Luftkissen wenn z.B. zwischen größeren kugelförmigen Erhebungen eine oder mehrere kleinere kugelförmige Erhebungen angeordnet sind. Dies verringert dann die Adhäsionserscheinung.

Außerdem sind derartige kugelförmige Erhebungen mit einem Radius in diesen Bereichen von Vorteil, da die einzelnen Fasern des Faserverbandes einen größeren Querschnitt aufweisen als die Erhebungen. Somit kann sich ein Luftpolster ausbilden, dass nicht oder nur kaum durch die einzelnen Fasern beeinflusst wird, da die Fasern nicht in die Zwischenräume der Erhebungen eindringen können. Ebenso werden die einzelnen Fasern durch die Erhebungen nicht beschädigt, da deren Kugelform keine scharfen Kanten aufweist. Die Fasern werden außerdem durch die im Vergleich zu den Fasern kleineren Erhebungen nicht abgeknickt, so dass die Fasern nicht abbrechen können.

Ein Weiterer zu erwähnender Vorteil ist, wenn die Mantelfläche einen Rauigkeitswert, ausgebildet durch die kugelförmigen Erhebungen, zwischen 0,5 und 80, insbesondere zwischen 10 und 60, aufweist. Dabei ist der Rauigkeitswert durch eine mittlere Rautiefe gegeben. Des Weiteren besteht ein Zusammenhang zwischen dem Rauigkeitswert und der Größe der kugelförmigen Erhebungen. Bei größeren kugelförmigen Erhebungen steigt im Allgemeinen auch der Rauigkeitswert.

Ein Verschwinden der Rauigkeit ist gleichbedeutend mit einer glatten Mantelfläche, was zu der nicht gewünschten Adhäsionserscheinung führt. Ein Ansteigen der Rauigkeit führt dann zu einer Vergrößerung des Luftkissens, welches wiederum zu einer sinkenden Adhäsionserscheinung führt.

Daneben ist es auch von Vorteil, wenn das Material geringer Wärmedehnung ein Invar-Material mit einem Wärmedehnungskoeffizienten von 0,55 bis 1,7 10e-6 1/K ist. Durch eine Reibung zwischen dem Faserverband und der Messwalze sowie durch die Maschine selbst wird Wärme erzeugt, die wiederum die Messwalze erwärmt. Dies führt zu einer Ausdehnung der Messwalze, wodurch ein Messen des Faserverbandes verfälscht wird. Durch das Invar-Material mit dem geringen Wärmedehnungskoeffizienten bleibt dieser Effekt jedoch so gering, dass dieser unter eine Toleranz der Messung fällt. Dabei ist das gängigste Invar-Material eine Legierung aus 64% Eisen und 36% Nickel. Es sind aber natürlich auch andere Materialien denkbar, die einen geringen Wärmedehnungskoeffizienten aufweisen.

Ein zusätzlicher Vorteil ist, wenn die Mantelfläche mit einer Oberflächenbeschichtung, wie zum Beispiel einer Chromschicht, beschichtet ist. Diese Oberflächenbeschichtung kann auch eine Rauigkeit aufweisen, wodurch ebenfalls die Adhäsionserscheinung verringert wird. Durch die Oberflächenbeschichtung wird dazu ein Verschleiß bei der Benutzung der Messwalze verringert, was sich kostensenkend auswirkt und die Wartungsintervalle verlängert.

Ganz besonders vorteilhaft ist, wenn ein Verhältnis der Größe der Wirkfläche zur Größe der Mantelfläche zwischen 15% und 85%, insbesondere zwischen 26,5% und 62,4%, ist. Dabei berechnet sich die Größe der Mantelfläche in bekannter Weise durch die Multiplikation aus Umfang und Breite der Messwalze. Die Breite der Messwalze ist dabei zwischen 5 mm und 30 mm, insbesondere etwa 15 mm. Die Wirkfläche berechnet sich dabei ebenfalls in bekannter Weise aus der Multiplikation von der Breite der Messwalze, dem Abstand der Rillen voneinander in Umfangsrichtung und deren Anzahl. Je größer dabei das Verhältnis ist, desto größer ist der Anteil der Wirkfläche. Bei einem kleineren Verhältnis ist der Anteil der Wirkfläche kleiner, was zu einem größeren Luftkissen führt. Ein größeres Luftkissen führt dabei wiederum zu einer geringeren Adhäsionserscheinung.

Zumindest eine der Messwalzen ist gemäß der vorangegangenen Beschreibung ausgebildet, wobei die genannten Merkmale einzeln oder in beliebiger Kombination, im Rahmen der Ansprüche, vorhanden sein können.

Für den Fall, dass beide Messwalzen mit derartigen Rillen, Bohrungen und/oder kugelförmige Erhebungen versehen sind, ist es besonders vorteilhaft, wenn im Betrieb der Vorrichtung jeweils die Berge (oder Täler) der beiden Messwalzen gegenüberliegen. D.h. es kommt ähnlich einem Zahnrad zu einer Zahn-auf-Zahn-Stellung. Anders als bei der Stellung Berg auf Tal, führt dies nicht zu einem Eindrücken des Faserverbandes durch die Berge in die Täler, was zu einer Toleranz und/oder Verfälschung beim Messen führt. Selbiges gilt natürlich auch für die Bohrungen oder den kugelförmigen Erhebungen.

Weitere Vorteile der Erfindung sind in den nachfolgenden Ausführungsbeispielen beschrieben. Es zeigt:
- **Figur 1**: Ausschnitt aus einem Querschnitt einer Messwalze,
- **Figur 2**: Ausschnitt aus einer Mantelfläche einer Messwalze mit sich kreuzenden Rillen,
- **Figur 3**: Ausschnitt aus einer Mantelfläche einer Messwalze mit Bohrungen,
- **Figur 4**: Ausschnitt aus einem Querschnitt einer Messwalze mit kugelförmigen Erhebungen, und
- **Figur 5**: Vorrichtung zum Messen eines Faserverbandes mit zwei Messwalze.

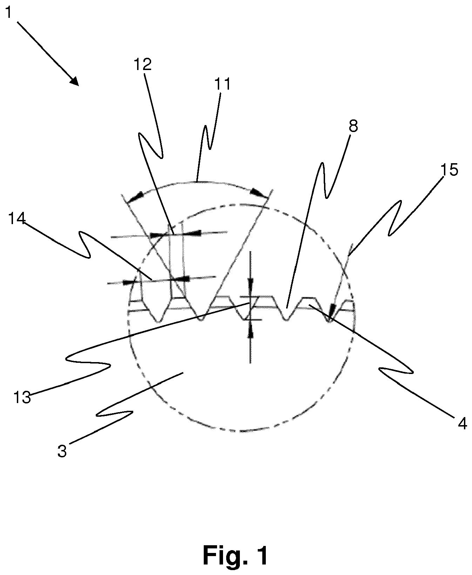

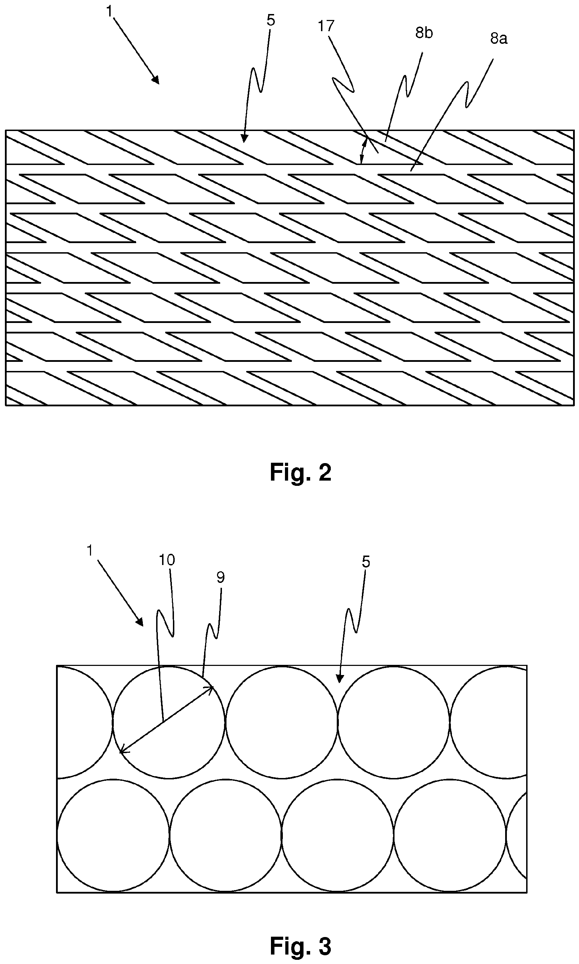

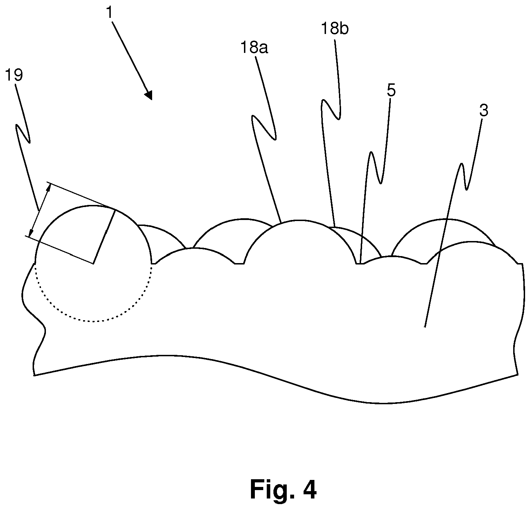

Figur 1 zeigt einen Ausschnitt aus einem Querschnitt einer Messwalze 1. Die Messwalze 1 besteht dabei aus einem Material geringer Wärmeausdehnung 3. Zusätzlich ist die Mantelfläche 5 der Messwalze 1 mit einer Oberflächenbeschichtung 4 beschichtet. In der Mantelfläche 5 sind Rillen 8 ausgebildet. Zusätzlich oder alternativ zu den Rillen 8 können aber auch Bohrungen 9, wie in Figur 3 und/oder kugelförmige Erhebungen 18 wie in Figur 4 beschrieben wird, ausgebildet sein.

Die Oberflächenbeschichtung 4 bedeckt dabei die Berge zwischen zwei Rillen 8 nur teilweise. Somit ist nur die Wirkfläche, die in Kontakt mit einem Faserverband 6 steht, beschichtet. Daneben ist aber auch denkbar, dass die Rillen 8 komplett mit der Oberflächenbeschichtung 4 beschichtet sind.

Die Rillen 8 weisen daneben einen Öffnungswinkel 11 auf. Jeweils zwei benachbarte Rillen 8 haben einen Abstand in Umfangsrichtung 12. Ebenso besitzen die Rillen 8 eine Rillentiefe 13 und eine Rillenbreite 14. Dabei weisen die Rillen 8 in ihrem Tal einen für die Fertigung durch z.B. Fräsen, typischen Radius 15 auf.

In Figur 2 ist ein Ausschnitt aus der Mantelfläche 5 der Messwalze 1 mit sich kreuzenden Rillen 8 gezeigt. Dabei ist ein erster Teil der Rillen 8a nur in axialer Richtung ausgerichtet. Ein zweiter Teil der Rillen 8b schneidet dabei den ersten Teil der Rillen 8a in einem Winkel 17. Da sich der Teil der Rillen 8a mit dem Teil der Rillen 8b unter dem Winkel 17 schneidet, ist eine Verbindung zwischen den jeweiligen Rillen 8a und 8b hergestellt. Ein Luftkissen kann nun über eine Rille 8b von einer der Rille 8a in eine danebenliegende Rille 8a gelangen.

Dabei ist es nicht zwingend, dass ein Teil der Rillen 8 axial ausgerichtet sind. Möglich ist auch, dass beide Teile der Rillen 8a und 8b schräg (sich aber immer noch in dem Winkel 17 schneiden) auf der Mantelfläche 5 der Messwalze 1 verlaufen. Dies führt zu jeweils zwei gegenläufigen Teilen der Rillen 8a und 8b.

In Figur 3 ist ein Ausschnitt aus der Mantelfläche 5 der Messwalze 1 mit Bohrungen 9 gezeigt. Dabei weisen die Bohrungen 9 einen Durchmesser 10 auf. Die gleichmäßige Ausrichtung der Bohrungen 9 ist dabei nur ein Beispiel. Ebenso wäre es denkbar, dass die Bohrungen verschiedene Durchmesser 10 aufweisen. Auch denkbar ist, dass die Bohrungen 9 in einer anderen Anordnung oder zufällig über die Mantelfläche 5 verteilt sind.

In Figur 4 ist ein Ausschnitt aus einem Querschnitt einer Messwalze 1 mit kugelförmigen Erhebungen 18 dargestellt. Die Messwalze 1 ist dabei aus einem Material geringer Wärmedehnung 3 ausgebildet. Auf der Mantelfläche 5 sind die kugelförmigen Erhebungen 18a und 18b angeordnet, wobei der Übersicht wegen nur diese beiden mit einem Bezugszeichen versehen sind. Die kugelförmige Erhebung 18a befindet sich dabei in dieser Figur im Vordergrund. Dahinter befindet sich eine kleinere kugelförmige Erhebung 18b. Wie zu erkennen ist, sind die kugelförmigen Erhebungen 18 in keinem Muster angeordnet, d.h. die Größen der kugelförmigen Erhebungen, die Ausrichtung und Abstände zwischen ihnen sind weitgehend beliebig. Möglich ist jedoch eine regelmäßige Ausrichtung, indem alle kugelförmigen Erhebungen die gleiche Größe aufweisen und gleichmäßig angeordnet sind. Die kugelförmigen Erhebungen weisen einen Radius 19 auf, wobei für ein leichteres Verständnis durch die gepunktete Linie (und die volle Linie) der volle Umkreis der kugelförmigen Erhebung angedeutet ist.

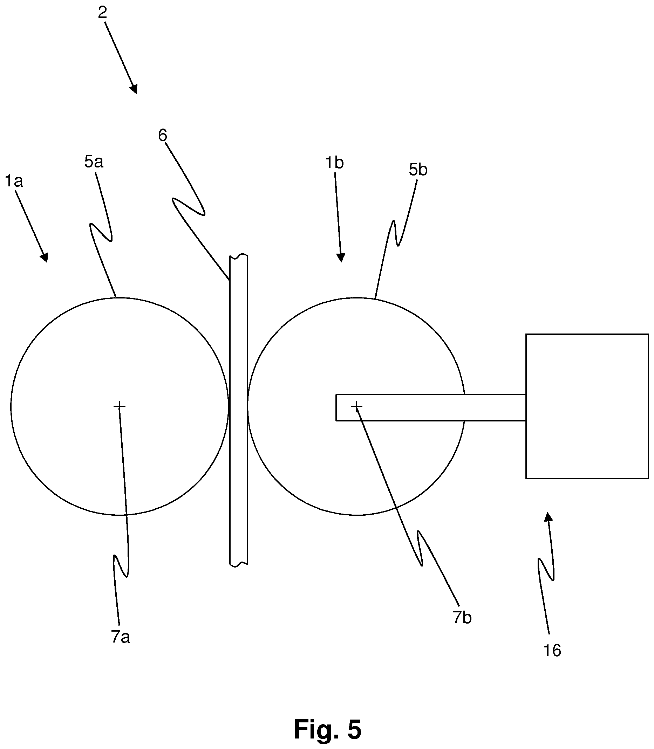

In Figur 5 ist eine Vorrichtung zum Messen eines Faserverbandes 2 mit zwei Messwalzen 1a und 1b gezeigt. Zu den Messwalzen 1a und 1b gehören die jeweiligen Mantelflächen 5a und 5b. Zwischen den Mantelflächen 5a und 5b ist ein Faserverband 6 hindurchgeführt. Die Messwalzen 5a und 5b sind um die jeweiligen Drehachsen 7a und 7b drehbar gelagert. Eine Anpressvorrichtung 16 übt einen Druck auf die Messwalze 5b aus, so dass sich der Abstand zwischen den Messwalzen 5a und 5b in Abhängigkeit der Dicke des Faserverbandes 6 verändert. Der Abstand der beiden Messwalzen 5a und 5b wird durch eine hier nicht gezeigte Anordnung aufgenommen. Dieser Abstand entspricht dann der Dicke des Faserverbandes 6.

Die vorliegende Erfindung ist nicht auf die dargestellten und beschriebenen Ausführungsbeispiele beschränkt. Abwandlungen im Rahmen der Patentansprüche sind ebenso möglich wie eine Kombination der Merkmale, auch wenn diese in unterschiedlichen Ausführungsbeispielen dargestellt und beschrieben sind.

### Bezugszeichenliste

- 1: Messwalze
- 2: Vorrichtung zum Messen eines Faserverbandes
- 3: Material geringer Wärmeausdehnung
- 4: Oberflächenbeschichtung
- 5: Mantelfläche
- 6: Faserverband
- 7: Drehachse
- 8: Rille
- 9: Bohrung
- 10: Durchmesser der Bohrung
- 11: Öffnungswinkel
- 12: Abstand in Umfangsrichtung
- 13: Rillentiefe
- 14: Rillenbreite
- 15: Radius
- 16: Anpressvorrichtung
- 17: Winkel
- 18: Kugelförmige Erhebung
- 19: Radius

## Patentansprüche

1. Messwalze zur Verwendung in einer Vorrichtung (2) zum Messen einer Dicke eines Faserverbandes (6), insbesondere an einer Strecke, Karde oder Kämmmaschine, aus einem Material geringer Wärmedehnung mit einer Oberflächenbeschichtung (4), und wobei die Messwalze (1) eine Mantelfläche (5) sowie eine Drehachse (7) aufweist, **dadurch gekennzeichnet, dass** die Mantelfläche (5) der Messwalze (1) eine Luftpolster bildende Struktur aus Rillen (8), Bohrungen (9) und/oder kugelförmigen Erhebungen (18) aufweist, dass die Rillen voneinander einen Abstand in Umfangsrichtung (12) von 0,25 mm bis 1,25 mm, insbesondere von 0,56 mm bis 0,85 mm, aufweisen, dass der Durchmesser der Bohrungen (10) auf der Mantelfläche (5) zwischen 0,1 mm und 1,25 mm, insbesondere zwischen 0,25 mm und 0,75 mm, und die Tiefe der Bohrungen zwischen 0,1 mm und 1,0 mm, insbesondere zwischen 0,2 mm und 0,45 mm, ist und/oder dass die kugelförmigen Erhebungen (18) einen Radius (19) zwischen 0,1 µm und 10 µm, insbesondere zwischen 0,6 µm und 4 µm, aufweisen.

2. Messwalze nach dem vorherigen Anspruch, **dadurch gekennzeichnet, dass** die Rillen (8) einen Öffnungswinkel (11) von 40° bis 105°, insbesondere von 60° bis 84°, eine Rillentiefe (13) von 0,1 mm bis 1,0 mm, insbesondere von 0,2 mm bis 0,45 mm, und/oder eine Rillenbreite (14) von 0,2 mm bis 1,0 mm, insbesondere von 0,41 mm bis 0,59 mm, aufweisen.

3. Messwalze nach einem oder mehreren der vorherigen Ansprüche, **dadurch gekennzeichnet, dass** die Anzahl der Rillen (8) in Umfangsrichtung zwischen 50 und 600, insbesondere zwischen 200 und 400, ist.

4. Messwalze nach einem oder mehreren der vorherigen Ansprüche, **dadurch gekennzeichnet, dass** sich zumindest ein Teil der Rillen (8) in einem Winkel (17) von 0° bis 90°, insbesondere in einem Winkel (17) von 16° bis 75°, schneiden.

5. Messwalze nach einem oder mehreren der vorherigen Ansprüche, **dadurch gekennzeichnet, dass** die Anzahl der Bohrungen (9) auf der Mantelfläche (5) zwischen 1000 und 25000, insbesondere zwischen 5000 und 15000, ist.

6. Messwalze nach einem oder mehreren der vorherigen Ansprüche, **dadurch gekennzeichnet, dass** die Mantelfläche einen Rauigkeitswert, ausgebildet durch die kugelförmigen Erhebungen (18), zwischen 0,5 und 80, insbesondere zwischen 10 und 60, aufweist.

7. Messwalze nach einem oder mehreren der vorherigen Ansprüche, **dadurch gekennzeichnet, dass** das Material geringer Wärmedehnung ein Invar-Material mit einem Wärmedehnungskoeffizienten von 0,55 10e-6 1/K bis 1,7 10e-6 1/K ist.

8. Messwalze nach einem oder mehreren der vorherigen Ansprüche, **dadurch gekennzeichnet, dass** ein Verhältnis der Größe einer Wirkfläche zur Größe der Mantelfläche (5) zwischen 15% und 85%, insbesondere zwischen 26,5% und 62,4%, ist.

9. Vorrichtung zum Messen einer Dicke eines Faserverbandes, mit zwei drehbar gelagerten Messwalzen (1a, 1b) mit Mantelflächen (5a, 5b), wobei die Drehachsen (7a, 7b) der Messwalzen (1a, 1b) parallel zueinander ausgerichtet sind, und dass der Faserverband (6) zwischen den Mantelflächen (5a, 5b) der Messwalzen (1a, 1b) hindurchführbar ist, und mit einer Anpressvorrichtung (16), mittels der der Abstand der Messwalzen (1a, 1b) zueinander veränderbar ist, und dass damit ein Druck auf den durch die Messwalzen (1a, 1b) eingeschlossenen Faserverband (6) aufbringbar ist, **dadurch gekennzeichnet, dass** zumindest eine der Messwalzen (1a, 1b) nach einem oder mehreren der vorherigen Ansprüche ausgebildet ist.

## Claims

1. Measuring roller for a device (2) for measuring the thickness of a fiber composite (6), in particular on a draw frame, a carding engine or a comber, made of a material of low thermal expansion and having a surface coating (4), and whereas the measuring roller (1) features a shell surface (5) as well as a rotary axis (7), **characterized in that** the shell surface (5) of the measuring roller (1) features a structure forming an air buffer, made of grooves (8), boreholes (9) and/or spherical projections (18), that the grooves feature among each other a gap in the circumferential direction (12) from 0.25 mm to 1.25 mm, in particular from 0.56 mm to 0.85 mm_that the diameter of the boreholes (10) at the shell surface (5) is between 0.1 mm and 1.25 mm, in particular between 0.25 mm and 0.75 mm, and the depth of the boreholes is between 0.1 mm and 1.0 mm, in particular between 0.2 mm and 0.45 mm, and/or that the spherical projections (18) feature a radius (19) of between 0.1 µm and 10 µm, in particular between 0.6 µm and 4 µm.

2. Measuring roller according to the preceding claim, **characterized in that** the grooves (8) feature an opening angle (11) from 40° to 105°, in particular from 60° to 84°, the grooves feature a groove depth (13) from 0.1 mm to 1.0 mm, in particular from 0.2 mm to 0.45 mm, and/or the groove features a groove width (14) from 0.2 mm to 1.0 mm, in particular from 0.41 mm to 0.59 mm.

3. Measuring roller according to one or more of the preceding claims, **characterized in that** the number of grooves (8) in the circumferential direction is between 50 and 600, in particular between 200 and 400.

4. Measuring roller according to one or more of the preceding claims, **characterized in that** at least a part of the grooves (8) intersects at an angle (17) of 0° to 90°, in particular at an angle (17) of 16° to 75°.

5. Measuring roller according to one or more of the preceding claims, **characterized in that** the number of boreholes (9) at the shell surface (5) is between 1,000 and 25,000, in particular between 5,000 and 15,000.

6. Measuring roller according to one or more of the preceding claims, **characterized in that** the shell surface features a roughness value, formed by the spherical projections (18), between 0.5 and 80, in particular between 10 and 60.

7. Measuring roller according to one or more of the preceding claims, **characterized in that** the material of low thermal expansion (3) is an Invar material with a thermal expansion coefficient of 0.55 10e-6 1/K to 1.7 10e-6 1/K.

8. Measuring roller according to one or more of the preceding claims, **characterized in that** the ratio of the size of the active surface to the size of the shell surface (5) is between 15% and 85%, in particular between 26.5% and 62.4%.

9. Device for measuring the thickness of a fiber composite with two rotatable mounted measuring rollers (1a, 1b) with shell surfaces (5a, 5b), whereas the rotary axes (7a, 7b) of the measuring rollers (1a, 1b) are aligned parallel to each other, and the fiber composite (6) can be passed between the shell surfaces (5a, 5b) of the measuring rollers (1a, 1b), and with a pressing device (16), by means of which the gap between the measuring rollers (1a, 1b) can be changed in relation to one another, and that pressure can thus be applied at the fiber composite (6) enclosed by the measuring rollers (1a, 1b), **characterized in that** at least one of the measuring rolls (1a, 1b) is formed according to one or more of the preceding claims.

## Revendications

1. Rouleau de mesure destiné à être utilisé dans un dispositif (2) pour mesurer l'épaisseur d'un ensemble (6) de fibres, en particulier sur une étireuse, une cardeuse ou une peigneuse, réalisé en un matériau à faible dilatation thermique avec un revêtement de surface (4), dans lequel le rouleau de mesure (1) présente une surface d'enveloppe (5) ainsi qu'un axe de rotation (7), **caractérisé en ce que** la surface d'enveloppe (5) du rouleau de mesure (1) présente une structure formant un matelas d'air et constituée de rainures (8), d'alésages (9) et/ou d'élévations sphériques (18), **en ce que** les rainures présentent entre elles un espacement dans la direction circonférentielle (12) de 0,25 mm à 1,25 mm, en particulier de 0,56 mm à 0,85 mm, **en ce que** le diamètre des alésages (10) sur la surface d'enveloppe (5) est compris entre 0,1 mm et 1,25 mm, en particulier entre 0,25 mm et 0,75 mm et la profondeur des alésages est comprise entre 0,1 mm et 1,0 mm, en particulier entre 0,2 mm et 0,45 mm, et/ou **en ce que** les élévations sphériques (18) ont un rayon (19) compris entre 0,1 µm et 10 µm, en particulier entre 0,6 µm et 4 µm.

2. Rouleau de mesure selon la revendication précédente, **caractérisé en ce que** les rainures (8) présentent un angle d'ouverture (11) de 40° à 105°, en particulier de 60° à 84°, une profondeur (13) de rainure de 0,1 mm à 1,0 mm, en particulier de 0,2 mm à 0,45 mm, et/ou une largeur (14) de rainure de 0,2 mm à 1,0 mm, en particulier de 0,41 mm à 0,59 mm.

3. Rouleau de mesure selon l'une quelconque ou plusieurs des revendications précédentes, **caractérisé en ce que** le nombre de rainures (8) dans la direction circonférentielle est compris entre 50 et 600, en particulier entre 200 et 400.

4. Rouleau de mesure selon l'une quelconque ou plusieurs des revendications précédentes, **caractérisé en ce qu'**une partie au moins des rainures (8) se croisent sous un angle (17) de 0° à 90°, en particulier sous un angle (17) de 16° à 75°.

5. Rouleau de mesure selon l'une quelconque ou plusieurs des revendications précédentes, **caractérisé en ce que** le nombre des alésages (9) sur la surface d'enveloppe (5) est compris entre 1 000 et 25 000, en particulier entre 5 000 et 15 000.

6. Rouleau de mesure selon l'une quelconque ou plusieurs des revendications précédentes, **caractérisé en ce que** la surface d'enveloppe présente une valeur de rugosité, formée par les élévations sphériques (18), comprise entre 0,5 et 80, en particulier entre 10 et 60.

7. Rouleau de mesure selon l'une quelconque ou plusieurs des revendications précédentes, **caractérisé en ce que** le matériau à faible dilatation thermique est un matériau invar avec un coefficient de dilatation thermique de 0,55 10e-6 1/K à 1,7 10e-6 1/K.

8. Rouleau de mesure selon l'une quelconque ou plusieurs des revendications précédentes, **caractérisé en ce qu'**un rapport entre la taille d'une surface active et la taille de la surface d'enveloppe (5) est compris entre 15 % et 85 %, en particulier entre 26,5 % et 62,4 %.

9. Dispositif pour mesurer l'épaisseur d'un ensemble de fibres, avec deux rouleaux de mesure (1a, 1b) montés de manière rotative avec des surfaces d'enveloppe (5a, 5b), dans lequel les axes de rotation (7a, 7b) des rouleaux de mesure (1a, 1b) sont orientés parallèlement l'un à l'autre, et l'ensemble de fibres (6) peut être guidé entre les surfaces d'enveloppe (5a, 5b) des rouleaux de mesure (1a, 1b), et avec un dispositif de pressage (16) au moyen duquel la distance des rouleaux de mesure (1a, 1b) l'un par rapport à l'autre peut être modifiée, et qu'une pression peut ainsi être appliquée sur l'ensemble de fibres (6) enfermé par les rouleaux de mesure (1a, 1b), **caractérisé en ce que** l'un au moins des rouleaux de mesure (1a, 1b) est réalisé selon l'une quelconque ou plusieurs des revendications précédentes.
